(19) **Europäisches Patentamt**
**European Patent Office**
**Office européen des brevets**

(11) **EP 1 871 178 B1**

(12) **FASCICULE DE BREVET EUROPEEN**

(45) Date de publication et mention de la délivrance du brevet:
**28.11.2012 Bulletin 2012/48**

(51) Int Cl.:
*A23K 1/175* (2006.01)       *A23K 1/18* (2006.01)

(21) Numéro de dépôt: **06743283.1**

(22) Date de dépôt: **11.04.2006**

(86) Numéro de dépôt international:
**PCT/EP2006/061530**

(87) Numéro de publication internationale:
**WO 2006/108845 (19.10.2006 Gazette 2006/42)**

(54) **ADDITIF ALIMENTAIRE NON MEDICAMENTEUX POUR ANIMAUX, ALIMENTS SUPPLEMENTES LE CONTENANT ET PROCEDE POUR AMELIORER LA CROISSANCE DES ANIMAUX**

NICHTARZNEIMITTELWIRKSAMER FUTTERZUSATZSTOFF FÜR TIERE, DIESEN ENTHALTENDE ANGEREICHERTE FUTTERMITTEL,UND VERFAHREN ZUR ERHÖHUNG DES TIERWACHSTUMS

NON-MEDICINAL FOOD ADDITIVE FOR ANIMALS, SUPPLEMENTED FOODS CONTAINING SAME AND METHOD FOR IMPROVING ANIMAL GROWTH

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **13.04.2005 FR 0503671**

(43) Date de publication de la demande:
**02.01.2008 Bulletin 2008/01**

(73) Titulaires:
• **Institut Regional des Materiaux Avances (IRMA)**
  **56270 Ploemeur (FR)**
• **INVIVO NSA**
  **56250 Saint Nolff (FR)**

(72) Inventeurs:
• **HAMON, Christian**
  **F-44600 Saint-Nazaire (FR)**
• **GUYONVARCH, Alain**
  **F-56000 Vannes (FR)**

(74) Mandataire: **Faivre Petit, Frédérique et al**
**Cabinet Regimbeau**
**20, rue de Chazelles**
**75847 Paris Cedex 17 (FR)**

• RAKIC V M ET AL: "CO interaction with zeolites studied by TPD and FTIR: transition-metal ion-exchanged FAU-type zeolites" MICROPOROUS AND MESOPOROUS MATERIALS, ELSEVIER SCIENCE PUBLISHING, NEW YORK, US, vol. 27, no. 1, janvier 1999 (1999-01), pages 27-39, XP004154502 ISSN: 1387-1811
• FORSTER H ET AL: "Investigations of coke deposits formed during deep oxidation of benzene over Pd and Cu exchanged Y-type zeolites" APPLIED CATALYSIS A: GENERAL, ELSEVIER SCIENCE, AMSTERDAM, NL, vol. 153, no. 1-2, 29 mai 1997 (1997-05-29), pages 31-41, XP004338108 ISSN: 0926-860X
• DATABASE WPI Section Ch, Week 200205 Derwent Publications Ltd., London, GB; Class B05, AN 2002-034962 XP002348349 & CN 1 310 945 A (CHEN S) 5 septembre 2001 (2001-09-05)
• PATENT ABSTRACTS OF JAPAN vol. 016, no. 176 (C-0934), 27 avril 1992 (1992-04-27) & JP 04 021517 A (NIPPON CHEM IND CO LTD; others: 01), 24 janvier 1992 (1992-01-24)
• PATENT ABSTRACTS OF JAPAN vol. 014, no. 280 (C-0729), 18 juin 1990 (1990-06-18) & JP 02 084957 A (SHINAGAWA NENRYO KK; others: 01), 26 mars 1990 (1990-03-26)

(56) Documents cités:
**EP-A1- 1 862 081        WO-A-03/072116**
**US-A- 5 314 852**

- Y. INOUE; M. HOSHINO; H. TAKAHASHI; T. NOGUSHI; T. MURATA; Y. KANZAKI; H. HAMASHIMA; M. SASATSU: "Bactericidal activity of Ag-zeolite mediated by reactive oxygen species under aerated conditions" JOURNAL OF INORGANIC CHEMISTRY, vol. 92, no. 1, 30 septembre 2002 (2002-09-30), pages 37-42, XP002394930
- MATSUMURA Y ET AL: "Mode of bactericidal action of silver zeolite and its comparison with that of silver nitrate" APPLIED AND ENVIRONMENTAL MICROBIOLOGY, WASHINGTON,DC, US, vol. 69, no. 7, juillet 2003 (2003-07), pages 4278-4281, XP002308887 ISSN: 0099-2240

- C.H. HU ET AL.: "Effects of copper-bearing montmorillonite on growth performance and digestive function of growing pigs" ASIAN-AUSTRALASIAN JOURNAL OF ANIMAL SCIENCES., vol. 17, no. 11, 2004, pages 1575-1581, XP008053679 KRSUWEON. cité dans la demande

**Description**

La présente invention concerne un additif alimentaire non médicamenteux promoteur de croissance des animaux, les aliments supplémentés le contenant, ainsi qu'un procédé pour améliorer la croissance des animaux.

Les additifs alimentaires « promoteurs de croissance » sont utilisés depuis longtemps, pour améliorer les performances zootechniques des animaux (mammifères, oiseaux ou poissons). Ce sont des produits obtenus soit par fermentation, soit par synthèse chimique, soit par extraction de plantes.

Jusqu'à présent les antibiotiques étaient utilisés de façon extensive en tant que promoteur de croissance. Toutefois, les réglementations ont évolué et sont devenues de plus en plus strictes et les antibiotiques seront sans doute interdits dans l'alimentation animale dans un futur proche.

Certains promoteurs de croissance non médicamenteux existent déjà.

Parmi les promoteurs de croissance, les argiles contenant du cuivre telles que la montmorillonite (argile feuilletée) ont été proposées (Xia et al., 2004 Poultry Science 83 :1868-1875, Xu et al., Asian-Aust. J. Anim. Sci. 2003. Vol 16, N°11 : 1673-1679, Xia et al., Asian-Aust. J. Anim. Sci. 2004. Vol 17, N°12 : 1712-1716 *et* Hu et al., Asian-Aust. J. Anim. Sci. 2004. Vol 17, N°11 : 1575-1581). Toutefois, les doses nécessaires à l'effet promoteur de croissance sont très élevées (1,5g/kg d'aliment, c'est-à-dire environ 1500 ppm).

Ceci pose un problème au niveau du coût de transport de ce promoteur ou de l'aliment l'incorporant. De plus, l'introduction de 1500 ppm de minéral inerte peut induire une séquestration de certains oligoéléments. En outre la teneur en cuivre de la montmorillonite échangée s'élève à 2,45 %, ce qui constitue un apport de cuivre dans la ration de 36,75 ppm, et excède la teneur en cuivre maximale autorisée par la réglementation CEE dans les aliments pour la plupart des espèces (cuivre constitutif des matières premières + cuivre apporté).

L'article de Rakic et al (Microporous and Mesoporous Materials 27 (1999) 27-39) décrit deux zéolithes pures à 99 % de type CuY et CuX et ayant respectivement les formules $(0,75/2)$ $Cu^{2+}.0,25Na^{+}.AlO_2.2,43SiO_2$ et $(0,97/2)Cu^{2+}.0,03Na^{+}.AlO_2.1,21SiO_2$. Ces zéolithes ne sont toutefois divulguées que dans le cadre de leur interaction avec le monoxyde de carbone. Ce document ne décrit aucune utilisation de ces zéolithes dans l'alimentation animale.

L'article de Becker et al (Applied Catalysis A : General 153 (1997) 31-41) décrit une zéolithe de type Y échangée au cuivre de formule $(0,19/2)$ $Cu^{2+}.0,81Na^{+}.AlO_2.2,5SiO_2$ contenant 2,5 % en poids de cuivre par rapport au poids total de la zéolithe.

Toutefois, un tel document ne concerne que les dépôts de cokes formés durant l'oxydation du benzène sur du palladium et des zéolithes. Ce document ne concerne pas l'alimentation des animaux.

L'abrégé de la demande de brevet chinoise CN 1310945 décrit un agent promoteur de croissance des animaux composé de 10 à 20 portions d'humate de sodium, de 2 à 5 portions de succinate de zinc, de 2 à 5 portions de sulfate de zinc, de 1 à 2 portions de sulfate de fer et de 64 à 80 portions de poudre de zéolithe. Cependant, la zéolithe utilisée n'est pas échangée avec un métal.

Le brevet japonais JP 02084957 décrit une zéolithe antibactérienne de formule $[(Ag_2O)x\,(M'O)_y\,(M''_2O)z]\,Al_2O_3\,mSiO_2nH_2O$. Une telle zéolithe contient obligatoirement un ion argent et un ion bivalent M' appartenant au groupe IIa, Ib ou au groupe IIb du tableau périodique. Elle n'a de plus pas la fonction de promouvoir la croissance des animaux.

La demande de brevet européen EP 1 862 081 décrit des compositions alimentaires pour animaux ayant une forte activité antimicrobienne et comprenant une zéolithe échangée à l'argent. Il n'est pas fait mention cependant de l'utilisation d'une zéolithe échangée au cuivre ou au zinc.

Il existe donc à ce jour un réel besoin d'un promoteur de croissance des animaux non médicamenteux qui puisse être actif à faible dose et donc aisément transportable.

Or, de façon surprenante, les inventeurs ont découvert qu'une zéolithe échangée au cuivre pouvait être utilisée en tant que promoteur de croissance chez les animaux à des doses bien inférieures à 1500 ppm.

Ainsi la présente invention concerne un additif alimentaire non médicamenteux, promoteur de croissance des animaux, contenant une zéolithe pure à 99 % (c'est-à-dire une zéolithe de synthèse) partiellement ou totalement échangée avec un cation $C^{m+}$ de formule générale I suivante

$$\underbrace{}_{m}\; yC^{m+}(1-y)\underbrace{}_{n} M^{n+}AlO_2xSiO_2$$

dans laquelle x est supérieur à 1, avantageusement compris entre 1 et 15, de façon avantageuse entre 1 et 10 ;

$M^{n+}$ représente un ion échangeable alcalin ou alcalino terreux, avantageusement $Na^+$, $K^+$, $Ca^{2+}$ ou $Li^+$, de façon avantageuse $Na^+$ ;

n est compris entre 1 et 2 ;

y est le taux d'échange et est compris entre 0,001 et 1 ;

$C^{m+}$ est un cation métallique choisi parmi le cuivre $Cu^{2+}$, ou le zinc $Zn^{2+}$, et étant avantageusement cuivre $Cu^{2+}$ ;

m est compris entre 1 et $2_2$.

à l'exception :

- de la zéolithe de type Y de formule $(0,75/2)\ Cu^{2+}.0,25Na^+.AlO_2.2,43\ SiO_2$,
- de la zéolithe de type X de formule $(0,97/2)\ Cu^{2+}.0,03Na^+.AlO_2.1,21\ SiO_2$, et
- de la zéolithe de type Y de formule $(0,19/2)\ Cu^{2+}.0,81Na^+.AlO_2.2,5\ SiO_2$.

Dans un mode de réalisation avantageux, y est compris entre 0,001 et 0,80, avantageusement entre 0,01 et 0,80, de façon avantageuse entre 0,1 et 0,80, avantageusement entre 0,1 et 0,75 ; de façon avantageuse entre 0,1 et 0,5.

Les zéolithes synthétiques, c'est-à-dire pures à environ 99 %, sont des silicates microporeux cristallisés dont les tailles des canaux et cavités varient selon la structure entre 3 et 13 Å. Ils se présentent sous forme de poudre pulvérulente, la taille des cristaux étant en moyenne de quelques microns, avantageusement comprise entre 1 et 2 microns.

x est le rapport Si/Al. A l'état normal, il y a de l'eau (condensation capillaire) dans les pores d'une zéolithe. On peut enlever l'eau par élévation de température. De par leur structure tétraédrique (enchaînement de tétraèdres $SiO_4$ et $AlO_4$ avec mise en commun des oxygènes, ce qui entraîne une charge négative sur chaque Aluminium ($AlO_2^-$), compensée par un cation $M^{n+}$), les zéolithes sont des échangeurs cationiques où l'on peut remplacer le cation $M^{n+}$ (le plus souvent $Na^+$ (n=1) après synthèse) par d'autres cations $C^{m+}$. Ces opérations d'échange et leur réalisation sont connues de l'homme de l'art.

D'une manière générale, pour réaliser ces échanges, la zéolithe est mise en suspension sous agitation dans une solution aqueuse d'un sel métallique dont on souhaite introduire le cation $C^{m+}$ ($Cu^{++}$ par exemple sous forme de sulfate (m=2)) par échange dans la zéolithe.

La réaction d'échange est gérée par la loi d'action de masse. En considérant une zéolithe sous sa forme sodique ($M^{n+}=Na^+$) avec échange par un cation $C^+$ (m=1), la réaction s'écrit :

$$Na^+ Z^- + C^+(S) \longleftrightarrow C^+Z^- + Na^+(S)$$

S : Solution

Z : Zéolithe

Cette réaction est équilibrée et la constante d'équilibre Kp ne dépend que de la température.

De par la notion d'équilibre, l'échange n'est le plus souvent pas total et il reste après échange à la fois $Na^+$ et $C^+$ dans les zéolithes qui se répartissent sur les différents sites cationiques.

On peut échanger aussi $M^{n+}$ par des cations bivalents (par exemple $C^{m+} = Cu^{++}$) ou trivalents mais dans ce cas, 1 $Cu^{++}$ remplace bien entendu 2$Na^+$ si $M^{n+} = Na^+$ (n=1) et $C^{m+} = Cu^{++}$ (m=2).

Les paramètres qui vont influencer l'échange et donc la teneur en cation $C^{m+}$ après échange seront la température, la concentration en sel métallique dans la solution et le rapport volume solution / poids (V/P) de zéolithe ; le temps intervient si l'on n'atteint pas l'équilibre.

Avantageusement, la zéolithe selon la présente invention est choisie parmi la zéolithe de type A, la zéolithe de type X, la zéolithe de type Y, la mordenite, la ferriérite, la zéolithe béta et les structures de type pentasil. Avantageusement, elle est choisie parmi la zéolithe de type A, la zéolithe de type X et la zéolithe de type Y, de façon avantageuse, il s'agit d'une zéolithe à structure faujasite, de façon encore plus avantageuse, il s'agit de la zéolithe de type Y.

En particulier pour une zéolithe de type A, x = 1. Avantageusement, la zéolithe de type A est choisie dans le groupe constitué par la zéolithe 3A, 4A ou 5A.

Avantageusement, pour les zéolithes de type X, x est égal à 1,25. De façon avantageuse, une zéolithe de type Y, x est égal à 2,6. De façon encore plus avantageuse, pour une mordenite, x est égal à 5,5. En particulier, pour une zéolithe de structure de type pentasil, x est égal à 13,5. Avantageusement, pour une férriérite, x est égal à 8,8.

De façon avantageuse, le cation métallique $C^{m+}$ de la zéolithe selon la présente invention est le cuivre $Cu^{2+}$.

Dans un autre mode de réalisation avantageux, la zéolithe selon la présente invention est de type Y, le cation métallique $C^{m+}$ est le cuivre $Cu^{2+}$ et la zéolithe contient entre 1 et 12,5 % en poids par rapport au poids total de la zéolithe de cuivre, avantageusement entre 3 et 9 %, de façon encore plus avantageuse entre 5 et 6 % en poids. De façon avantageuse l'ion $M^{n+}$ est le $Na^+$.

Dans un mode de réalisation particulier de l'invention, la zéolithe selon la présente invention est de type Y, le cation métallique $C^{m+}$ est le zinc $Zn^{2+}$, et la zéolithe contient entre 1 et 13 % en poids de zinc par rapport au poids total de la zéolithe, de préférence entre 3 et 8 % en poids de zinc par rapport au poids total de la zéolithe, avantageusement environ 5 % en poids de zinc.

Avantageusement, l'additif alimentaire selon la présente invention est destiné à l'alimentation des animaux de rente ou de compagnie, avantageusement choisi parmi les porcins (en particulier les porcs), les bovins, les ovins, les caprins, les volailles (en particulier les poulets ou les dindes), les lapins, les poissons et les oiseaux.

Dans la plupart des cas et selon le but, le promoteur de croissance est administré par voie orale aux animaux. En sélectionnant les formes individuelles d'administration, les caractéristiques spécifiques de chaque espèce ainsi que l'âge des animaux doit être pris en compte.

De plus, il doit être certain en pratique que chaque animal reçoit la dose nécessaire de promoteur de croissance et qu'aucune perte évitable n'a lieu.

Les aliments contenant le promoteur de croissance peuvent être indifféremment présentés sous toutes les formes habituelles connues en élevage.

Les aliments peuvent ainsi être des aliments simples ou composés, complets ou complémentaires (oligoéléments, enzymes, acidifiants, substances aromatiques et apéritives, vitamines,...).

Dans le cas où les jeunes animaux sont toujours nourris par leur mère, le promoteur de croissance est préférentiellement injecté directement dans la gorge sous forme d'une suspension ou de soluté.

Pour les veaux, le promoteur de croissance peut être administré sous la forme d'une suspension laitière. L'incorporation dans l'eau de boissons est également possible. Pour des animaux qui consomment déjà de la nourriture solide, le promoteur de croissance peut être mélangé avec la nourriture. Suivant les espèces animales, cette nourriture peut être choisi parmi les grains de céréales, produits et sous produits ; les graines et fruits oléagineux, leurs produits et sous produits ; les graines de légumineuses, leurs produits et sous produits ; les tubercules et racines, leurs produits et sous produits ; les autres graines et fruits, leurs produits et sous produits ; les fourrages, y compris les fourrages grossiers ; les autres plantes, leurs produits et sous produits ; les produits laitiers ; les produits d'animaux terrestres ; le poisson, les autres animaux marins, leurs produits et sous produits ; les minéraux ; et les vitamines, seuls ou en mélange.

Une forme préférée d'administration est sous la forme de cubes ou de granules qui contiennent en dehors du promoteur de croissance des composants habituels de l'alimentation de l'animal en question qui peuvent être choisis parmi les grains de céréales, produits et sous produits ; les graines et fruits oléagineux, leurs produits et sous produits ; les graines de légumineuses, leurs produits et sous produits ; les tubercules et racines, leurs produits et sous produits ; les autres graines et fruits, leurs produits et sous produits ; les fourrages, y compris les fourrages grossiers ; les autres plantes, leurs produits et sous produits ; les produits laitiers ; les produits d'animaux terrestres ; le poisson, les autres animaux marins, leurs produits et sous produits ; les minéraux ; et les vitamines, seuls ou en mélange.

La composition peut être administrée aux poissons sous forme de capsules ayant un diamètre de 1 à 7 mm qui sont insolubles dans l'eau à température ambiante. Une autre possibilité est l'administration de granulés de nourriture contenant de la graisse dans lesquels le promoteur de croissance est insoluble ou peu soluble.

Les doses d'incorporation du promoteur de croissance peuvent varier selon l'espèce, l'âge, le niveau d'ingestion des animaux et, dans une certaine mesure, selon l'effet recherché. Il appartiendra au spécialiste, à l'aide d'essais systématiques, de déterminer pour chaque usage la dose optimale. Avantageusement, dans le cadre de la présente invention, la zéolithe selon la présente invention est présente en une quantité comprise entre 5 et 200 ppm par rapport au poids total de l'aliment, avantageusement entre 5 et 100 ppm, de façon avantageuse entre 5 et 80 ppm, de façon encore plus avantageuse entre 5 et 20 ppm, avantageusement égale à environ 10 ppm.

Avantageusement, les recommandations pour l'administration de la zéolithe selon la présente invention sont de 0,8 à 1,2 mg / kg de poids vif (PV) x jour pour l'espèce porcine, et de 0,6 à 0,9 mg / kg PV x jour pour les volailles.

Ainsi, l'additif alimentaire « promoteur de croissance » selon la présente invention peut se présenter sous forme pure ou en mélange avec divers supports et/ou autres additifs admissibles.

Avantageusement, en raison du faible taux d'incorporation nécessaire pour obtenir l'effet promoteur de croissance, la zéolithe selon la présente invention n'est pas incorporée en l'état dans l'aliment, mais par l'intermédiaire d'un prémélange d'additifs. La présente invention concerne donc un prémélange d'additif alimentaire non médicamenteux promoteur de croissance des animaux caractérisé en ce qu'il contient un additif alimentaire selon la présente invention sur un support et/ou en combinaison avec au moins un autre additif alimentaire des animaux. Cet autre additif alimentaire peut être non médicamenteux et/ou présenter des effets de promotion de croissance (on peut citer les acidifiants, les extraits végétaux, les substances aromatiques, les facteurs de croissance, seul ou en mélanges).

Ce prémélange peut donc être :

(1) - spécifique : uniquement zéolithe selon la présente invention sur support ad hoc, tels que par exemple les coproduits de céréales, le carbonate de calcium, les rafles de maïs, les autres argiles, seuls ou en mélange ;

(2) - partiellement spécifique : zéolithe selon la présente invention + 1 ou 2 autres additifs à effets comparables à ceux de la zéolithe tels que par exemple les acidifiants, les extraits végétaux, les substances aromatiques, les facteurs de croissance, seul ou en mélanges, incorporé à des doses inférieures à leurs doses efficaces, sur support tels que par exemple les coproduits de céréales, le carbonate de calcium, les rafles de maïs, les autres argiles, seuls ou en mélange ;

(3) - non spécifique : zéolithe selon la présente invention incorporée à un prémix complet contenant au moins des vitamines et des oligoéléments.

Dans tous les cas de figures, le prémélange est incorporé dans l'aliment final distribué aux animaux avantageusement à des taux variant le plus souvent de 500 g à 5 kg de prémélange par tonne d'aliment.

La présente invention concerne en outre un aliment supplémenté pour animaux, contenant un additif alimentaire selon la présente invention ou un prémélange selon la présente invention.

Avantageusement, l'aliment supplémenté pour animaux selon la présente invention est tel que la zéolithe est présente en une quantité de 5 à 200 ppm en poids par rapport au poids total de l'aliment, avantageusement entre 5 et 100 ppm, de façon avantageuse entre 5 et 80 ppm, de façon encore plus avantageuse entre 5 et 20 ppm, avantageusement égale à environ 10 ppm en poids.

Enfin, la présente invention concerne un procédé pour améliorer la croissance des animaux caractérisé en ce qu'il consiste à incorporer dans la nourriture desdits animaux une zéolithe que définie précédemment ou une zéolithe choisie parmi :

- la zéolithe de type Y de formule $(0,75/2)\ Cu^{2+}.0,25Na^+.AlO_2.2,43\ SiO_2$,
- la zéolithe de type X de formule $(0,97/2)\ Cu^{2+}.0,03Na^+.A1O_2.1,21\ SiO_2$, et
- la zéolithe de type Y de formule $(0,19/2)\ Cu^{2+}.0,81Na^+.AlO_2.2,5\ SiO_2$,
- avantageusement en une quantité de 5 à 200 ppm en poids par rapport au poids total des aliments, avantageusement entre 5 et 100 ppm, de façon avantageuse entre 5 et 80 ppm, de façon encore plus avantageuse entre 5 et 20 ppm, avantageusement égale à environ 10 ppm en poids.

Dans un mode de réalisation avantageux, le procédé selon la présente invention est tel que la zéolithe selon la présente invention est incorporée sous forme d'additif alimentaire selon la présente invention ou sous forme de prémélange.

Avantageusement, les animaux sont choisis parmi les animaux de rente ou de compagnie, avantageusement parmi les porcins (en particulier les porcs), les bovins, les ovins, les caprins, les volailles (en particulier les poulets ou les dindes), les lapins, les poissons et les oiseaux.

Les effets bénéfiques de cet additif alimentaire peuvent se résumer comme suit (sur les mammifères, les oiseaux, les poissons) :

- augmenter la croissance des animaux ;
- et/ou abaisser leur indice de consommation (c'est-à-dire la quantité d'aliment nécessaire à une unité de gain de poids), et corollairement, augmenter leur indice de transformation (c'est-à-dire le gain de poids permis par unité d'aliment).

Les exemples suivants illustrent l'invention sans en limiter la portée.

Description des figures

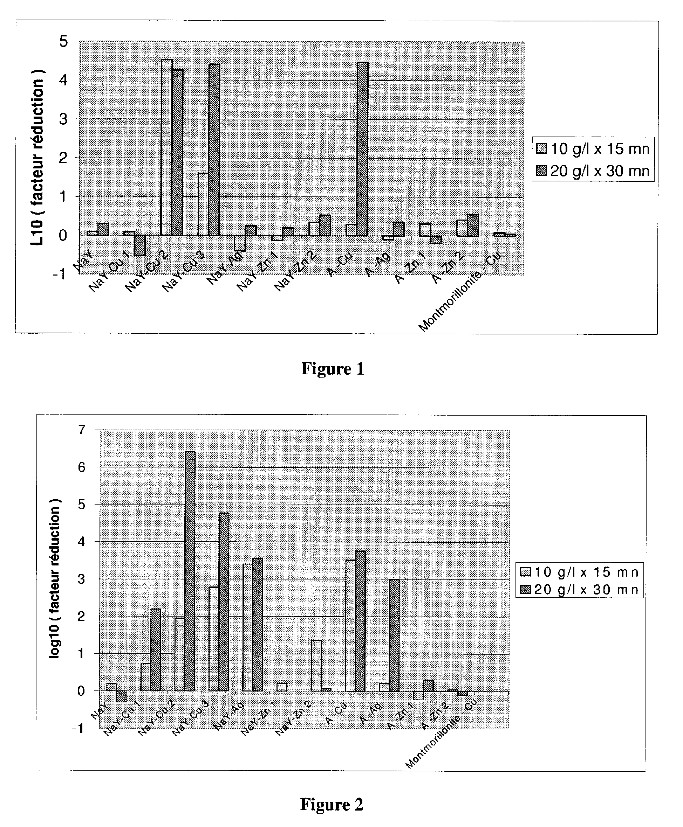

La figure 1 représente l'activité inhibitrice d'*Escherichia Coli* pour différentes zéolithes échangées ou non avec du cuivre, de l'argent ou du zinc et pour la montmorillonite échangée au cuivre (doses de 10 grammes par litre de zéolithe pour une exposition de 15 minutes ou de 20 grammes par litre de zéolithe pour une exposition de 30 minutes). La figure 2 représente l'activité inhibitrice de *Clostridium Sporogenes* pour différentes zéolithes échangées ou non avec du cuivre, de l'argent ou du zinc et la montmorillonite échangée au cuivre à des doses de 10 grammes par litre de zéolithe pour une exposition de 15 minutes ou de 20 grammes par litre de zéolithe pour une exposition de 30 minutes.

## EXEMPLE 1 : Préparation d'une zéolithe de type Y contenant 5,1 % en poids de $Cu^{2+}$ selon la présente invention

La zéolithe NaY de départ (avant échange) utilisée a un rapport Si/Al (atomique) de 2,6 (x = 2,6); sa formule générale est la suivante $Na\ AlO_2\ 2,6\ SiO_2\ wH_2O$, l'eau représentant l'eau absorbée (condensation capillaire) dans les pores de la zéolithe, que l'on peut éliminer en élevant la température. L'ouverture des pores est comprise entre 8 et 9 Å avec une supercage à 13 Å.

On procède à l'échange de $Na^+$ par $Cu^{++}$ de la manière suivante :

La zéolithe NaY sous forme de poudre est mise en suspension sous agitation dans une solution aqueuse (eau déminéralisée) de nitrate de cuivre $Cu^{++}$ (d'autres sels tel que sulfate peuvent convenir).

Dans le cas présent, on utilise 200 g de zéolithe dans 0,5 litre de solution aqueuse de sulfate de cuivre (0,44 Molaire), soit un rapport V/P de 2,5, une température de 70 °C et un temps d'échange de 3 heures.

On récupère ensuite la zéolithe échangée, qui présente une couleur bleuâtre, par filtration et lavage sur entonnoir filtrant et on lave sur filtre en percolation avec deux litres d'eau déminéralisée. La zéolithe est ensuite séchée (à l'étuve à 120 °C pendant une nuit) et la teneur en cuivre mesurée par ICP (spectroscopie d'émission par torche plasma : inducted conducted plasma (sur sec à 400 °C)) est de 5,2 %. Le % d'échange est donc de 40 %.

**EXEMPLES 2 A 10: Préparation d'une zéolithe de type Y ou A échangée au cuivre ou, au zinc selon la présente invention ou à l'argent (exemple comparatif)**

On peut faire varier les conditions opératoires et donc la teneur en cuivre. Le tableau 1 suivant représente les différentes conditions opératoires pour les exemples 2 à 10 et les zéolithes selon la présente invention avec la teneur en métal obtenue.

Pour tous ces exemples : V/P = 2,5, la température d'échange est de 60 °C et le temps d'échange est de 3 heures.

| Exemple | Zéolithe de départ | Sel Métallique | CONCENTRATION (Molaire) en sel métallique dans la solution aqueuse | Teneur Métal en % en poids | % d'échange |
|---------|-------------------|----------------|-----------------------------------------------------------------|---------------------------|-------------|
| 2 | NaY | Sulfate Cuivre | 0,05 | 2,35 % Cu | 18 |
| 3 | NaY | Sulfate Cuivre | 1 | 8,7 % Cu | 68 |
| 4 | NaY | Nitrate Zinc | 0,6 | 5 % Zn | 37 |
| 5* | NaY | Nitrate Argent | 0,01 | 0,2 % Ag | 0,5 |
| 6* | NaY | Nitrate Argent | 0,5 | 14,7 % Ag | 37 |
| 7 | A | Sulfate Cuivre | 0,5 | 9 % Cu | 41 |
| 8 | A | Nitrate Zinc | 0,6 | 5 % Zn | 22 |
| 9* | A | Nitrate Argent | 0,01 | 0,2 % Ag | 0,3 |
| 10* | A | Nitrate Argent | 0,5 | 12,5 % Ag | 18 |
| * exemples comparatifs | | | | | |

**EXEMPLE 11 : Préparation d'une zéolithe de type Y contenant 3,3 % en poids de cuivre selon la présente invention**

On peut réaliser l'échange à l'état solide entre la zéolithe et le sel métallique.

Ainsi, on mélange intimement dans un mortier 100 g de zéolithe avec 13 g de sulfate de cuivre Cu $SO_4$ $5H_2O$. Le mélange est conservé ainsi pendant une nuit à température ambiante.

On procède ensuite à un lavage par de l'eau déminéralisée (1 litre) sur entonnoir filtrant pour extraire le cuivre non échangé. La zéolithe est ensuite séchée à 120 °C ; la teneur en cuivre (mesurée par ICP) est de 3,3 % en poids ; le taux d'échange est de 25 %.

On renouvelle une opération identique à 60 °C (au lieu de la température ambiante) ; la teneur en cuivre est sensiblement la même, ce qui est logique car le cuivre est dans ce cas pratiquement échangé en totalité.

**EXEMPLE 12: Tests in vivo des zéolithes selon la présente invention NaY contenant entre 5 et 6% en poids de cuivre chez différents animaux**

Les données de croissance et d'indice de consommation (qui illustre l'efficacité alimentaire, comme étant la quantité d'aliment nécessaire pour obtenir un kilogramme de poids vif supplémentaire - par définition, l'indice de consommation est sans unité, puisque le rapport de 2 mesures équivalentes) sont indiquées sous forme indicée : le témoin est indicé à 100, et les performances des lots expérimentaux avec zéolithe sont indicés par rapport à cette base. Un indice de 103,2 signifie une performance supérieure de 3,2 % à celle du témoin.

**Porcelets**

Premier essai porcelet: 192 porcelets entre 42 et 70 jours d'âge allottés selon leur poids vif, et recevant un aliment correspondant à leur stade physiologique, supplémenté de quantités variables de zéolithe NaY contenant 6 % en poids de cuivre selon la présente invention (0 ppm (témoin) 3, 6 ou 12 ppm).

Les performances moyennes sont rassemblées dans le tableau 2 suivant :

| | témoin | | | |
|---|---|---|---|---|
| Taux d'incorporation de zéolithe selon la présente invention en ppm | 0 | 3 | 6 | 12 |
| Croissance | 100 | 94,7 | 109 | 103,5 |
| Indice conso | 100 | 103,2 | 95,7 | 95,7 |

L'incorporation de zéolithe selon la présente invention permet une amélioration de la croissance des porcelets entre 42 et 70 jours d'âge. Cette amélioration est modélisable selon une équation du $2^e$ degré, qui admet un maximum pour un taux d'incorporation de la zéolithe selon la présente invention compris entre 9 et 10 ppm par rapport au poids total de l'aliment.

Deuxième essai porcelet : 56 porcelets de 21 à 42 jours d'âge ($1^{er}$ âge) allottés selon leur poids vif, et recevant un aliment correspondant à leur stade physiologique, supplémenté de quantités variables de zéolithe NaY contenant 6 % en poids de cuivre selon la présente invention (0 ppm (témoin) 5, 10 ou 20 ppm).

Les performances moyennes sont rassemblées dans le tableau 3 suivant :

| | témoin | | | |
|---|---|---|---|---|
| Taux d'incorporation de zéolithe selon la présente invention en ppm | 0 | 5 | 10 | 20 |
| Croissance | 100 | 114,6 | 116,4 | 110,4 |

L'incorporation de zéolithe selon la présente invention permet une amélioration de la croissance des porcelets entre 21 et 42 jours d'âge. Cette amélioration est modélisable selon une équation du 2e degré, qui admet un maximum pour un taux d'incorporation de la zéolithe selon la présente invention d'environ 12 ppm par rapport au poids total de l'aliment.

Troisième essai porcelet : 56 porcelets de 21 à 42 jours d'âge, puis 42 à 70 jours d'âge allottés selon leur poids vif, et recevant un aliment correspondant à leur stade physiologique, supplémenté de quantités variables de zéolithe NaY contenant 6 % en poids de cuivre selon la présente invention (2,8 ppm (témoin) 5, 7,2 ou 11,6 ppm).

Les performances moyennes sont rassemblées dans le tableau 4 suivant :

| | témoin | | | |
|---|---|---|---|---|
| Taux d'incorporation de zéolithe selon la présente invention en ppm | 2,8 | 5 | 7,2 | 11,6 |
| Croissance | 100 | 105,5 | 109,7 | 108,9 |

L'incorporation de zéolithe selon la présente invention permet une amélioration de la croissance des porcelets entre 21 et 69 jours d'âge. Cette amélioration est modélisable selon une équation du 2e degré, qui admet un maximum pour un taux d'incorporation de la zéolithe selon la présente invention compris entre 9 et 10 ppm par rapport au poids total de l'aliment.

**Poulets**

660 poulets de 1 à 28 jours d'âge allottés selon leur poids vif, et recevant un aliment correspondant à leur stade physiologique, supplémenté de quantités variables de zéolithe NaY contenant 6 % en poids de cuivre selon la présente invention (0 ppm (témoin) 5, 10 ou 20 ppm).

Les performances moyennes sont rassemblées dans le tableau 5 suivant :

| | Témoin | | |
|---|---|---|---|
| Taux d'incorporation de zéolithe selon la présente invention en ppm | 0 | 6 | 15 |
| Croissance | 100 | 105,4 | 104,5 |

L'incorporation de zéolithe selon la présente invention permet une amélioration faible de la croissance des poulets entre 1 et 28 jours d'âge.

**Dindons**

360 dindons de 28 à 55 jours d'âge allottés selon leur poids vif, et recevant un aliment correspondant à leur stade physiologique, supplémenté de 5 ppm de zéolithe NaY contenant 6 % en poids de cuivre selon la présente invention ou de 0 ppm (témoin).

Les performances moyennes sont rassemblées dans le tableau 6 suivant :

| | Poids J28 | Poids J55 | Gain de poids | Consommation période | Indice consommation |
|---|---|---|---|---|---|
| | gramme | gramme | gramme | gramme | |
| Témoin | 990 | 3077 | 2087 | 4357 | 2,09 |
| Zéolithe selon la présente invention 5 ppm | 996 | 3235 | 2239 | 4440 | 1,97 |
| Sign.statistique | | p<0,001 | p<0,001 | | p<0,001 |

L'incorporation de zéolithe selon la présente invention permet une amélioration très hautement significative de la croissance des dindons entre 28 et 55 jours d'âge.

**EXEMPLE 13 : Test in vitro des zéolithes sur différents microorganismes**

La méthodologie utilisée est basée sur la mesure de l'activité bactéricide de la zéolithe selon la présente invention en milieu liquide, et est comparable quel_que soit le microorganisme testé. Le principe est le suivant :

- préparation d'une suspension de bactéries à $10^8$ ou $10^9$ germes par ml
- mise en contact de 50 ml de cette suspension avec une certaine quantité de zéolithe selon la présente invention et agitation pendant un temps donné
- numération des germes restant après traitement.

Chaque couple dose x temps comporte son propre témoin.
Les résultats sont exprimés en facteur de réduction de la colonie initiale (nombre de germes du témoin sur nombre de germes après traitement).

**Intérêt, spécificité de l'ion métallique et spécificité de la zéolithe**

L'activité réductrice moyenne a été calculée comme le quotient de la réduction moyenne, exprimée en log 10, par la valeur moyenne de l'exposition (dose x temps).
Différentes zéolithes ont été testées :

Une zéolithe non échangée (NaY) (exemple comparatif), des zéolithes NaY échangées au cuivre contenant 2,3 % en poids de cuivre selon l'exemple 2 (NaY-CuI : taux d'échange 18 %), 8,7 % en poids de cuivre selon l'exemple 3 (NaY-Cu2: taux d'échange 68 %) et 10 % en poids de cuivre (NaY-Cu3 : taux d'échange 78 %), une zéolithe NaY échangée à l'argent contenant 14,7 % en poids d'argent selon l'exemple comparatif 6 (NaY-Ag : taux d'échange 37 %), des zéolithes NaY échangées au zinc contenant 5,2 % en poids de Zinc (NaY-Zn1 : taux d'échange 38 %) et 5 % en poids de zinc selon l'exemple 4 (NaY-Zn2 : taux d'échange 37 %), une zéolithe A échangée au cuivre contenant 8,9 % en poids de cuivre (A-Cu : taux d'échange 41 %), une zéolithe A échangée à l'argent contenant 12,5 % en poids d'argent selon l'exemple comparatif 10 (A-Ag : taux d'échange 18 %), des zéolithes A échangées au zinc contenant 8,4 % en poids de zinc (A-Znl : taux d'échange 38 %) et 8 % en poids de zinc (A-Zn2 : taux d'échange 36 %), ainsi que la montmorillonite échangée au cuivre (exemple comparatif) faisant l'objet des publications chinoises (Xia et al., 2004 Poultry Science 83 :1868-1875, Xu et al., Asian-Aust. J. Anim. Sci. 2003. Vol 16, N°11 : 1673-1679, Xia et al., Asian-Aust. J. Anim. Sci. 2004. Vol 17, N°12 : 1712-1716 et Hu et al., Asian-Aust. J. Anim. Sci. 2004. Vol 17, N°11: 1575-1581).

Les figures 1 et 2 illustrent sur *E.Coli* et *C.S porogenes* l'activité réductrice moyenne de ces zéolithes. Il est ainsi à noter :

- l'absence d'activité de la zéolithe native (NaY vs NaY -Cu)
- l'activité intermédiaire de NaY - Ag (échange à l'argent)
- l'activité de la zéolithe A échangée au cuivre ou à l'argent
- la très faible activité de la montmorillonite échangée au cuivre.

**Revendications**

1.  Additif alimentaire non médicamenteux, promoteur de croissance des animaux, contenant une zéolithe pure à 99 % partiellement ou totalement échangée avec un cation $C^{m+}$ de formule générale I suivante

$$\underline{1}\, yC^{m+}(1-y)\underline{1}\, M^{n+}AlO_2xSiO_2$$
$$m \qquad\qquad n$$

dans laquelle x est supérieur à 1, avantageusement compris entre 1 et 15;
$M^{n+}$ représente un ion échangeable alcalin ou alcalino terreux, avantageusement choisi parmi $Na^+$, $K^+$, $Ca^{2+}$ ou Li ;
n est compris entre 1 et 2 ;
y est le taux d'échange et est compris entre 0,001 et 1 ;
$C^{m+}$ est un cation métallique choisi parmi le cuivre $Cu^{2+}$ ou le zinc $Zn^{2+}$
m est compris entre 1 et 2,
à l'exception

- de la zéolithe de type Y de formule $(0,75/2)\ Cu^{2+}.0,25Na^+.AlO_2.2,43\ SiO_2$
- de la zéolithe de type X de formule $(0,97/2)\ Cu^{2+}.0,03Na^+.AlO_2.1,21\ SiO_2$ et
- de la zéolithe de type Y de formule $(0,19/2)\ Cu^{2+}.0,81Na^+.AlO_2.2,5\ SiO_2$

2.  Additif selon la revendication 1 **caractérisé en ce que** y est compris entre 0,01 et 0,80, avantageusement entre 0,1 et 0,80.

3.  Additif selon l'une quelconque des revendications précédentes, **caractérisé en ce que** la zéolithe est choisie parmi la zéolithe de type A, la zéolithe de type X, la zéolithe de type Y, la mordenite, la ferriérite, la zéolithe béta et les structures de type pentasil, avantageusement il s'agit d'une zéolithe à structure faujasite, de façon avantageuse, il s'agit de la zéolithe de type Y.

4.  Additif selon l'une quelconque des revendications précédentes **caractérisé en ce que** le cation métallique $C^{m+}$ est le cuivre $Cu^{2+}$.

5.  Additif selon l'une quelconque des revendications 1 à 4 **caractérisé en ce que** la zéolithe est de type Y, **en ce que** le cation métallique $C^{m+}$ est le cuivre $Cu^{2+}$ et **en ce que** la zéolithe contient entre 1 et 12,5 % en poids par rapport au poids total de la zéolithe de cuivre, avantageusement entre 3 et 9 %, de façon encore plus avantageuse entre 5 et 6% en poids.

6.  Additif alimentaire selon l'une quelconque des revendications 1 à 3 **caractérisé en ce que** le cation métallique $C^{m+}$ est le zinc $Zn^{2+}$, **en ce que** la zéolithe est de type Y et **en ce que** la zéolithe contient entre 1 et 13 % en poids de zinc par rapport au poids total de la zéolithe, de préférence entre 3 et 8 % en poids de zinc par rapport au poids total de la zéolithe, avantageusement environ 5 % en poids de zinc.

7. Additif alimentaire selon l'une quelconque des revendications précédentes **caractérisé en ce qu'**il est destiné à l'alimentation des animaux de rente ou de compagnie, avantageusement choisis parmi les porcins, les bovins, les ovins, les caprins, les volailles, les lapins, les poissons et les oiseaux.

8. Prémélange d'additif alimentaire non médicamenteux promoteur de croissance des animaux **caractérisé en ce qu'**il contient un additif alimentaire selon l'une quelconque des revendications 1 à 7 sur un support et/ou en combinaison avec au moins un autre additif alimentaire des animaux, avantageusement choisi parmi les additifs non médicamenteux promoteur de croissance des animaux et/ou des vitamines et oligoéléments.

9. Aliment supplémenté pour animaux, **caractérisé en ce qu'**il contient un additif alimentaire selon l'une quelconque des revendications 1 à 7 ou un prémélange selon la revendication 8.

10. Aliment supplémenté pour animaux selon la revendication 9, **caractérisé en ce que** la zéolithe est présente en une quantité de 5 à 200 ppm en poids par rapport au poids total de l'aliment, avantageusement égale à environ 10 ppm en poids.

11. Procédé pour améliorer la croissance des animaux **caractérisé en ce qu'**il consiste à incorporer dans la nourriture desdits animaux une zéolithe telle que définie dans l'une quelconque des revendications 1 à 7 ou une zéolithe choisie parmi

- la zéolithe de type Y de formule $(0,75/2)$ $Cu^{2+}.0,25Na^+.A10_2.2,43$ $SiO_2$,
- la zéolithe de type X de formule $(0,97/2)$ $Cu^{2+}.0,03Na^+.AlO_2.1,21$ $SiO_2$ et
- la zéolithe de type Y de formule $(0,19/2)$ $Cu^{2+}.0,81Na^+.AlO_2.2,5$ $SiO_2$,

avantageusement en une quantité de 5 à 200 ppm en poids par rapport au poids total des aliments.

12. Procédé selon la revendication 11 **caractérisé en ce que** la zéolithe est incorporée sous forme d'additif alimentaire selon l'une quelconque des revendications 1 à 7 ou sous forme de prémélange selon la revendication 8.

13. Procédé selon la revendication 11 ou 12 **caractérisé en ce que** les animaux sont choisis parmi les porcins, les bovins, les ovins, les caprins, les volailles, les lapins, les poissons et les oiseaux.

**Claims**

1. Non-medical food additive that is an animal growth promoter containing 99% pure zeolite partially or totally exchanged with a $C^{m+}$ cation with the general formula I below:

$$\frac{1}{m}yC^{m+}(1-y)\frac{1}{n}M^{n+}AlO_2xSiO_2$$

in which x is greater than 1 and advantageously between 1 and 15;
$M^{n+}$ represents an alkaline or alkaline earth exchangeable ion, advantageously $Na^+$, $K^+$, $Ca^{2+}$ or Li;
n is between 1 and 2;
y is the exchange rate and is between 0.001 and 1;
$C^{m+}$ is a metallic cation chosen from copper $Cu^{2+}$ or zinc $Zn^{2+}$;
m is between 1 and 2,
with the exception of:

- type Y zeolite of formula $(0.75/2)$ $Cu^{2+}.0.25$ $Na^+.AlO_2.2.43$ $SiO_2$,
- type X zeolite of formula $(0.97/2)$ $Cu^{2+}.0.03$ $Na^+.AlO_2-1.21$ $SiO_2$ and
- type Y zeolite of formula $(0.19/2)$ $Cu^2.0.81$ $Na^+.AlO_2.2.5$ $SiO_2$.

2. Additive according to claim 1, **characterized in that** y is between 0.01 and 0.80, advantageously between 0.1 and 0.80.

3. Additive according to any one of the preceding claims, **characterized in that** the zeolite is chosen from among type A zeolite, type X zeolite, type Y zeolite, mordenite, ferrierite, beta zeolite and pentasil type structures, advantageously it is a zeolite with a faujasite structure and even better it is type Y zeolite.

4. Additive according to any one of the preceding claims, **characterized in that** the $C^{m+}$ metallic cation is copper $Cu^{2+}$.

5. Additive according to any one of claims 1 to 4, **characterized in that** the zeolite is of type Y, the metallic cation $C^{m+}$ is copper $Cu^{2+}$ and the zeolite contains between 1 and 12.5% by weight of copper as a proportion of the total weight of zeolite, advantageously between 3 and 9% and even better between 5 and 6% by weight.

6. Food additive according to any one of claims 1 to 3, **characterized in that** the metallic cation $C^{m+}$ is zinc $Zn^{2+}$, the zeolite is of type Y and the zeolite contains between 1 and 13% by weight of zinc as a proportion of the total weight of zeolite, preferably between 3 and 8% by weight of zinc as a proportion of the total weight of zeolite, and advantageously about 5% by weight of zinc.

7. Food additive according to any one of the preceding claims, **characterized in that** it is for use for feeding farm animals or pets, advantageously chosen from among porcines, bovines, ovines, goats, poultry, rabbits, fish and birds.

8. Premix of non-medical growth promoter food additive for animals, **characterized in that** it contains a food additive according to any one of claims 1 to 7 on a support and/or in combination with at least another animal food additive, advantageously chosen from among animal non-medical growth promoter additives and/or vitamins and trace elements.

9. Supplemented food for animals, **characterized in that** it contains a food additive according to any one of claims 1 to 7 or a premix according to claim 8.

10. Supplemented food for animals according to claim 9, **characterized in that** the quantity of zeolite present is 5 to 200 ppm by weight as a proportion of the total weight of the food, advantageously equal to about 10 ppm by weight.

11. Method of improving the growth of animals, **characterized in that** it consists of incorporating in the food of said animals a zeolite as that defined in any one of claims 1 to 7 or a zeolite chosen from among:

   - type Y zeolite of formula $(0.75/2)\ Cu^{2+}.0.25\ Na^{+}.AlO_2.2.43\ SiO_2$,
   - type X zeolite of formula $(0.97/2)\ Cu^{2+}.0.03\ Na^{+}.AlO_2.1.21\ SiO_2$ and
   - type Y zeolite of formula $(0.19/2)\ Cu^{2+}.0.81\ Na^{+}.\ AlO_2.2.5\ SiO_2$,

   advantageously in a quantity of 5 to 200 ppm by weight as a proportion of the total weight of the foods.

12. Method according to claim 11, **characterized in that** the zeolite is incorporated in the form of a food additive according to any one of claims 1 to 7 or in the form of a premix according to claim 8.

13. Method according to either claim 11 or 12, **characterized in that** the animals are chosen from among porcines, bovines, ovines, goats, poultry, rabbits, fish and birds.

**Patentansprüche**

1. Nicht-medikamentöser, das Wachstum von Tieren fördernder Futtermittelzusatzstoff, welcher einen 99 % reinen Zeolithen enthält, in dem ein teilweiser oder vollständiger Austausch mit einem Kation $C^{m+}$ der folgenden allgemeinen Formel I vorliegt

$$\underline{1}\ yC^{m+}(1-y)\underline{1}\ M^{n+}AlO_2xSiO_2$$
$$m \qquad\qquad n$$

worin x größer als 1 ist und vorteilhafterweise zwischen 1 und 15 beträgt;
$M^{n+}$ ein austauschbares alkalisches oder erdalkalisches Ion, welches vorteilhafterweise aus $Na^{+}$, $K^{+}$ $Ca^{2+}$ oder Li

ausgewählt ist, darstelle;

n zwischen 1 und 2 beträgt;

y der Austauschgrad ist und zwischen 0,001 und 1 beträgt;

$C^{m+}$ ein metallisches Kation ist, welches aus Kupfer $Cu^{2+}$ oder Zink $Zn^{2+}$ ausgewählt ist;

m zwischen 1 und 2 beträgt,

ausgenommen

- der Zeolith vom Y-Typ der Formel

$(0{,}75/2)Cu^{2+}.0{,}25Na^{+}.AlO_2.2{,}43\ SiO_2,$

- der Zeolith vom X-Typ der Formel

$(0{,}97/2)Cu^{2+}.0{,}03Na^{+}.AlO_2.1{,}21\ SiO_2$ und

- der Zeolith vom Y-Typ der Formel

$(0{,}19/2)Cu^{2+}.0{,}81Na^{+}.AlO_2.2{,}5\ SiO_2.$

2. Zusatzstoff gemäß Anspruch 1, **dadurch gekennzeichnet, dass** y zwischen 0,01 und 0,8, vorteilhafterweise zwischen 0,1 und 0,80, beträgt.

3. Zusatzstoff gemäß irgendeinem der vorhergehenden Ansprüche, **dadurch gekennzeichnet, dass** der Zeolith aus Zeolith vom A-Typ, Zeolith vom X-Typ, Zeolith vom Y-Typ, Mordenit, Ferrierit, Beta-Zeolith und Strukturen vom Pentasil-Typ ausgewählt ist, und es sich vorteilhafterweise um einen Zeolithen mit Faujasit-Struktur, bevorzugt um einen Zeolithen vom Y-Typ, handelt.

4. Zusatzstoff gemäß irgendeinem der vorhergehenden Ansprüche, **dadurch gekennzeichnet, dass** das metallische Kation $C^{m+}$ Kupfer $Cu^{2+}$ ist.

5. Zusatzstoff gemäß irgendeinem der Ansprüche 1 bis 4, **dadurch gekennzeichnet, dass** der Zeolith vom Y-Typ ist, dass das metallische Kation $C^{m+}$ Kupfer $Cu^{2+}$ ist, und dass der Zeolith zwischen 1 und 12,5 Gew.-%, bezogen auf das Gesamtgewicht des Zeolithen, bevorzugt zwischen 3 und 9 %, bevorzugter zwischen 5 und 6 Gew.-%, Kupfer enthält.

6. Futtermittelzusatzstoff gemäß irgendeinem der Ansprüche 1 bis 3, **dadurch gekennzeichnet, dass** das metallische Kation $C^{m+}$ Zink $Zn^{2+}$ ist, dass der Zeolith vom Y-Typ ist, und dass der Zeolith zwischen 1 und 13 Gew.-% Zink, bezogen auf das Gesamtgewicht des Zeolithen, bevorzugt zwischen 3 und 8 Gew.-% Zink, bezogen auf das Gesamtgewicht des Zeolithen, vorteilhafterweise etwa 5 Gew.-% Zink, enthält.

7. Futtermittelzusatzstoff gemäß irgendeinem der vorhergehenden Ansprüche, **dadurch gekennzeichnet, dass** er zur Fütterung von Nutztieren oder Haustieren, die vorteilhafterweise aus Schweinen, Rindern, Schafen, Ziegen, Geflügel, Kaninchen, Fischen und Vögeln ausgewählt sind, bestimmt ist.

8. Vormischung eines nicht-medikamentösen, das Wachstum von Tieren fördernden Futtermittelzusatzstoffs, **dadurch gekennzeichnet, dass** sie einen Futtermittelzusatzstoff gemäß irgendeinem der Ansprüche 1 bis 7 auf einem Träger und/oder in Kombination mit wenigstens einem anderen Futtermittelzusatzstoff für Tiere, welcher vorteilhafterweise aus nicht-medikamentösen, das Wachstum von Tieren fördernden Zusatzstoffen und/oder Vitaminen und Oligoelementen ausgewählt ist, enthält.

9. Supplementiertes Futtermittel für Tiere, **dadurch gekennzeichnet, dass** es einen Futtermittelzusatzstoff gemäß irgendeinem der Ansprüche 1 bis 7 oder eine Vormischung gemäß Anspruch 8 enthält.

10. Supplementiertes Futtermittel für Tiere gemäß Anspruch 9, **dadurch gekennzeichnet, dass** der Zeolith in einer Menge von 5 bis 200 Gew.-ppm, bezogen auf das Gesamtgewicht des Futtermittels, vorteilhafterweise mit etwa 10 Gew.-ppm, vorliegt.

11. Verfahren zur Verbesserung des Wachstums von Tieren, **dadurch gekennzeichnet, dass** es in der Beimengung eines Zeolithen, wie in irgendeinem der Ansprüche 1 bis 7 definiert, oder eines Zeolithen, der aus

- dem Zeolithen vom Y-Typ der Formel $(0,75/2)Cu^{2+}.0,25Na+.AlO_2.2,43\ SiO_2$,
- dem Zeolithen vom X-Typ der Formel $(0,97/2)Cu^{2+}.0,03Na+.AlO_2.1,21\ SiO_2$ und
- dem Zeolithen vom Y-Typ der Formel $(0,19/2)\ Cu^{2+}.\ 0,81Na^{+}.AlO_2.2,5\ SiO_2$

ausgewählt ist, vorteilhafterweise in einer Menge von 5 bis 200 Gew.-ppm, bezogen auf das Gesamtgewicht der Futtermittel, zu dem Futter der Tiere besteht.

12. Verfahren gemäß Anspruch 11, **dadurch gekennzeichnet, dass** der Zeolith in Form eines Futtermittelzusatzstoffs gemäß irgendeinem der Ansprüche 1 bis 7 oder in Form einer Vormischung gemäß Anspruch 8 beigemengt wird.

13. Verfahren gemäß Anspruch 11 oder 12, **dadurch gekennzeichnet, dass** die Tiere aus Schweinen, Rindern, Schafen, Ziegen, Geflügel, Kaninchen, Fischen und Vögeln ausgewählt sind.

**Figure 1**

**Figure 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 1310945
- JP 02084957 A
- EP 1862081 A

**Littérature non-brevet citée dans la description**

- **XIA et al.** *Poultry Science,* 2004, vol. 83, 1868-1875
- **XU et al.** *Asian-Aust. J. Anim. Sci.,* 2003, vol. 16 (11), 1673-1679
- **XIA et al.** *Asian-Aust. J. Anim. Sci.,* 2004, vol. 17 (12), 1712-1716
- **HU et al.** *Asian-Aust. J. Anim. Sci.,* 2004, vol. 17 (11), 1575-1581
- **RAKIC et al.** *Microporous and Mesoporous Materials,* 1999, vol. 27, 27-39
- **BECKER et al.** *Applied Catalysis A : General,* 1997, vol. 153, 31-41